Europäisches Patentamt

European Patent Office

Office européen des brevets

(11) Veröffentlichungsnummer: **0 075 735**
**B1**

(12) # EUROPÄISCHE PATENTSCHRIFT

(45) Veröffentlichungstag der Patentschrift:
26.02.86

(51) Int. Cl.⁴: **A 61 M 29/02**

(21) Anmeldenummer: **82107920.9**

(22) Anmeldetag: **28.08.82**

(54) **Vorrichtung zur Dilatation von Blutgefässen.**

(30) Priorität: **29.09.81 DE 3138623**

(43) Veröffentlichungstag der Anmeldung:
**06.04.83 Patentblatt 83/14**

(45) Bekanntmachung des Hinweises auf die Patenterteilung:
**26.02.86 Patentblatt 86/9**

(84) Benannte Vertragsstaaten:
**AT BE CH DE FR GB IT LI LU NL SE**

(56) Entgegenhaltungen:
**DE - A - 2 021 515**
**DE - A - 2 320 014**
**FR - A - 2 290 917**
**FR - A - 2 406 935**
**US - A - 4 271 839**
**US - A - 4 273 128**
**US - A - 4 284 073**

**MEDICAL PROGRESS TECHNOLOGY Band
8, Nr. 1, 1980, Berlin J.-C. FARCOT et al. " New
catheter-pump system for diastolic synchronized
coronary sinus retroperfusion " Seiten 29 bis 37**

(73) Patentinhaber: **Kuhl, Adolf, Dr. med. Ing.-grad.,
Finkenhof 3b, D-3000 Hannover 61 (DE)**

(72) Erfinder: **Kuhl, Adolf, Dr. med. Ing.-grad., Finkenhof 3b,
D-3000 Hannover 61 (DE)**

(74) Vertreter: **Selting, Günther, Dipl.-Ing. et al,
Deichmannhaus am Hauptbahnhof, D-5000 Köln 1 (DE)**

**Beschreibung**

Die Erfindung betrifft eine Vorrichtung zur Dilatation von Blutgefäßen, mit einem in ein Blutgefäß einsetzbaren, durch Druck aufweitbaren Dilatationskatheter und einem an dem Dilatationskatheter anschließbaren, ein Druckfluid enthaltenen Druckbehälter, dessen Druck zeitlich gesteuert veränderbar ist und der eine Vorrichtung zur Änderung des das Druckfluid enthaltenen Behältervolumens aufweist, welche zur Erzeugung von Druckimpulsen derart betätigbar ist, daß der Dilatationskatheter abwechselnd das Blutgefäß aufweitet und für den Blutdurchgang freigibt.

Es ist bekannt, Blutgefäße und andere Hohlräume im Körper mit Hilfe von aufblasbaren Kathetern zu öffnen oder zu erweitern oder auch zu verschließen. Dabei wird ein schlauchförmiger Katheter aus elastischem Material in das Blutgefäß bzw. die Körperhöhle eingeführt. Der Katheter ist mit einem ein Druckfluid enthaltenen Druckbehälter verbunden.

US—A—4 271 839 und US—A—4 273 128 beschreiben Dilatationskatheter, bei denen der Druck zum Aufblasen eines am Katheterende angeordneten Ballons gesteuert werden kann, um ein Blutgefäß aufzuweiten. Dies geschieht nach US—A—4 271 839 durch eine Pumpe, die impulsartig durch Hin- und Herbewegen eines Kolbens in einem Druckbehälter von Hand betrieben wird, womit eine Volumenveränderung im Druckbehälter bewirkt wird und daher Druckimpulse erzeugt werden.

Aus US—A—4 284 073 ist ein Katheter zum Pumpen des Blutes innerhalb eines Blutgefäßes bekannt, der durch eine impulsweise betriebene Pumpe intermittierend aufgepumpt wird, wobei sich der Katheter zwischen den Impulsen jeweils wieder entspannt. Als mögliche Pumpenantriebe werden hier elektromagnetische Erregermaschinen oder elektrische Schrittmotoren vorgeschlagen. Die Frequenz, mit der sich das Aufpumpen und Entspannen des Katheters wiederholt, ist dabei von der der Herztätigkeit abgeleitet und ist höher als diese.

Bei den bekannten Kathetern zur Aufweitung verengte Blutgefäße erfolgt die Aufweitung nur durch quasistationäre Drücke, und zwar immer dann, wenn der Katheter mit dem von außen einstellbaren Druck beaufschlagt wird. Die Folge davon sind relativ lange Behandlungszeiten bzw. geringe Wirkung der Behandlung.

Der Erfindung liegt die Aufgabe zugrunde, eine Vorrichtung der eingange genannten Art derart weiterzubilden, daß die Dilatationswirkung verbessert wird.

Zur Lösung dieser Aufgabe ist erfindungsgemäß vorgesehen, daß den Druckimpulsen ein in seiner Amplitude kleinerer, sich periodisch verändernder Wechseldruck überlagert ist.

Die den Druckimpulsen überlagerten Wechseldrücke haben zusätzliche Volumenänderungen und damit Kraftänderungen im Gefäß zur Folge, und beschleunigen die Dilatationswirkung. Derartige Wechseldrücke können beispielsweise sinusförmigen, rechteckförmigen oder dreieckigen Verlauf haben.

Eine noch stärkere Wirksamkeit des Dilatationsprozesses kann sich ergeben, wenn die Amplitude der höherfrequenten Schwingung während jedes Impulses der Impulserzeugerschaltung von einem Anfangswert bis zu einem Endwert zunimmt. Die Gefäßwand wird dabei zunehmend gedehnt und vor impulsartiger Erweiterung geschont.

Gemäß einer bevorzugten Ausführungform der Erfindung ist die Vorrichtung zur Änderung des Behältervolumens in Abhängigkeit von EKG-Impulsen des Patienten gesteuert. Hierdurch kann erreicht werden, daß das Dilatationselement nur während der Systole (Zusammenziehung des Herzens) für die Dauer etwa eines Drittels der gesamten Herzaktion aufgepumpt ist. Alternativ kann das Dilatationselement während eines oder mehrerer Herzzyklen dauernd aufgepumpt sein, um sich anschließend für eine bestimmte Zeitland zusammenzuziehen. Es ist auch möglich, die Erweiterung nur bei jedem zweiten, dritten, vierten usw. Zyklus durchzuführen. Bei bestimmten Gefäßen, z.B. bei periphären Gefäßen, wird der Katheter während der Diastole aufgeblasen und während der Systole entspannt, da die Perfusion in der Peripherie während der Systole höher ist.

Die Impulserzeugerschaltung kann einen Zähler aufweisen, der jeden n-ten Impuls unterdrückt, wobei n eine ganze Zahl ist, die größer ist als 1. Durch geeignete Wahl der Zahl n kann in Abhängigkeit von dem jeweiligen Einsatzort des Dilatationskatheters das zeitliche Verhältnis "Aufweitung: Durchblutung" festgelegt werden.

Gemäß einer weiteren bevorzugten Ausführungsform der Erfindung weist die Impulserzeugerschaltung einen Zähler auf. der jeweils über mehrere Ansteuerimpulse hinweg einen Ausgangsimpuls erzeugt, wobei die Ausgangsimpulse durch Impulslücken voneinander getrennt sind. Hierbei findet über mehrere Pulsschläge hinweg eine stetige Dilatation statt, die von Durchblutungsphasen unterbrochen wird, wobei die Dauer der Dilatationsphase nach Ort und Art des jeweiligen Gefäßes eingestellt werden können.

Gemäß einer vorteilhaften Weiterbildung der Erfindung ist vorgesehen, daß die von einem EKG abgeleitete Spannung einer nur auf die QRS-Gruppe ansprechenden Schwellwertschaltung zugeführt wird und daß der Schwellwertschaltung eine Verzögerungsschaltung mit einstellbarer Verzögerung nachgeschaltet ist. Die QRS-Gruppe ist im EKG durch einen Impuls besonders großer Amplitude gekennzeichnet. Dieser Impuls, der leicht erkannt werden kann wird zur Steuerung der Druckbeaufschlagung benutzt. Durch entsprechende Einstellung der Verzögerungsschaltung kann diejenige Phase, zwischen zwei QRS-Gruppen ausgewählt werden, in der die Gefäßdilatation erfolgt.

Um Gefäßschädigungen durch zu große Aufweitung des Katheters zu vermeiden, weist der Behälter ein Überdruckventil auf, das bei einem höchstzulässigen Druck im Innern des Katheters auslöst.

Im folgenden wird unter Bezugnahme auf die Zeichnungen ein Ausführungsbeispiel der Erfindung näher erläutert.

Es zeigen:

Figur 1 eine schematische Darstellung des Steuerteils der Vorrichtung,

Figur 2 Längsschnitte des Katheters im entspannten und im aufgepumpten Zustand,

Figur 3 Druckimpulse, jeweils mit überlagertem Wechseldruck, und

Figur 4 ein Impulsdiagramm für zwei unterschiedliche Betriebsarten der Vorrichtung nach Figur 1.

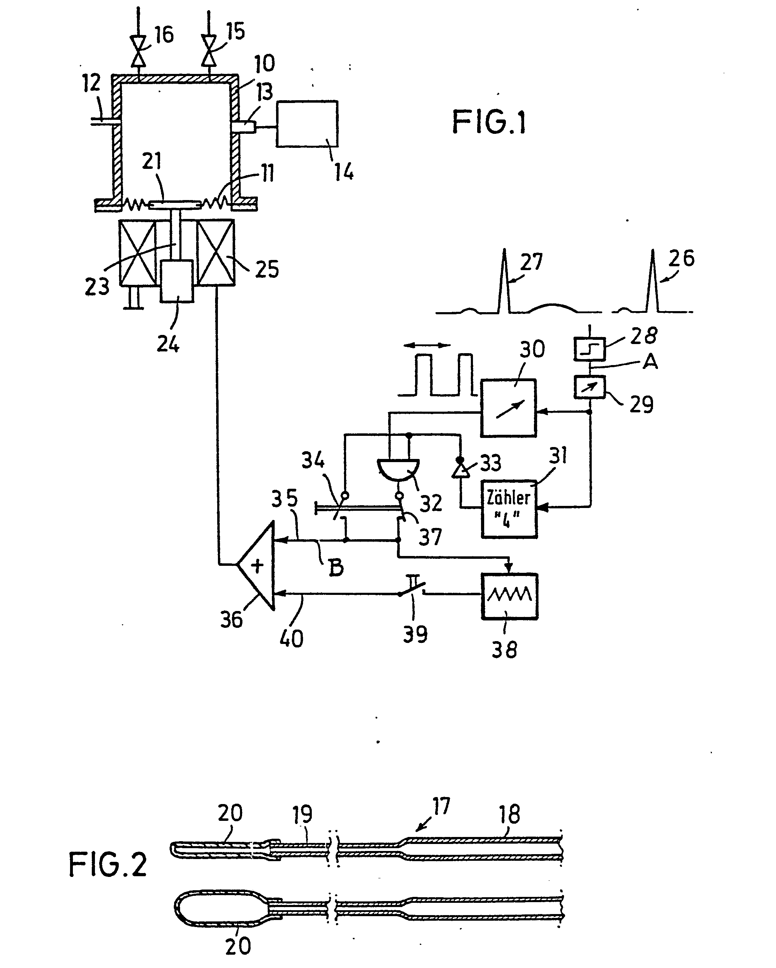

Gemäß Figur 1 ist ein Druckbehälter 10 vorgesehen, der an seiner Vorderwand mit einem Balg 11 abgedichtet ist. Der Druckbehälter 10 weist einen Katheteranschluß 12 und einen Druckmeßanschluß 13, an den ein Druckaufnehmer 14 angeschlossen ist, auf. Außerdem ist der Druckbehälter mit einem bei einem bestimmten Druck im Behälterinnern ansprechenden Überdruckventil 15 und einem manuell zu betätigenden Entlüftungsventil 16 ausgestattet.

Der Druckbehälter 10 und der angeschlossene Katheter sind mit einem Druckfluid, vorzugsweise einer inkompressiblen Flüssigkeit, gefüllt. Durch Eindrücken des Balges 11 in das Behälterinnere wird der Druck im Behälter und im Innern des Katheters vergrößert. Der in Figur 2 dargestellte Katheter 17, dessen distales Ende mit dem Anschluß 12 verbunden ist, besteht aus einem Schlauch 18, der an seinem proximalen Ende einen Abschnitt 19 verringerten Durchmessers aufweist. Der Schlauch 18 besteht aus einem zwar biegsamen, aber durch Druck nicht wesentlich aufweitbaren Material. Das vordere Ende des Schlauches 18 ist offen und hieran ist der Dilatationsteil 20 befestigt, der aus einem Material besteht, das wesentlich elastischer ist als dasjenige des Schlauches 18. Der Dilatationsteil 20 erweitert sich bei Druckbeaufschlagung des Katheters ballonartig wie in der unteren Darstellung von Figur 2 angegeben ist. Da der Schlauch 18 sich infolge eines Innendrucks nur ganz unbedeutend aufweitet, erfolgt die Volumenvergrößerung des Katheters nahezu ausschließlich im Bereich des Dilatationsteiles 20.

Die Druckbeaufschlagung erfolgt durch Bewegung des Balges 11 in das Innere des luftfrei mit Flüssigkeit gefüllten Behälters 10. Der Mittelbereich des Balges 11 ist an einer Platte 21 befestigt, von der eine Stange 23 nach außen absteht. Die Stange 23 ragt in eine Magnetspule 25 hinein. An ihrem Ende ist ein Eisenblock 24 befestigt, der aus dem rückwärtigen Ende der Magnetspule 25 herausragt. Wenn die Magnetspule 25 stromdurchflossen ist, wird der Eisenblock 24 in die Spule hineingezogen, wodurch die Platte 21 mit dem Balg 11 in das Innere des Druckbehälters 10 verschoben wird. Dadurch wird Druckflüssigkeit aus dem Druckbehälter 10 in den Katheter 17 transportiert, wodurch sich der Dilationsteil 20 aufweitet. Die Druckübertragung erfolgt weitgehend trägheitsfrei, so daß auch kurze Druckstöße der Platte 21 praktisch verzögerungslos auf den Dilatationsteil 20 übertragen werden. Anstelle des Balges 11 mit der Platte 21 kann auch ein anderes Bewegungssystem, z.B. eine Membran, ein Kolben o.dgl. benutzt werden. Der Antrieb erfolgt vorzugsweise elektromechanisch.

Die Steuerung der Magnetspule 25 geschieht durch die in Figur 1 dargestellte Schaltung in Abhängigkeit von den Impulsen des Elektrokardiogramms (EKG) 26 des Patienten. Hierzu wird der nadelartige Impuls 27 der QRS-Gruppe (Kammeranfangsgruppe) festgestellt. Dies geschieht durch eine Schwellwertschaltung 28, der die EKG-Spannung 26 zugeführt wird. Die Schwellwertschaltung 28 filtert ausschließlich die Spannungsspitzen 27 heraus. Ihr Ausgang ist an ein Verzögerungsglied 29 mit einstellbarer Verzögerung angeschlossen. Das Ausgangssignal der Verzögerungsschaltung 29 wird einer Impulsformerstufe 30 und einem Zähler 31 zugeführt. Die Impulsformerstufe 30 liefert Impulse konstanter Amplitude, wobei die Impulsdauer einstellbar ist. Ihr Ausgang ist an einen Eingang eines UND-Tores 32 angeschlossen.

Der Zähler 31 ist so ausgebildet, daß er jeweils bis zu einer bestimmten einstellbaren Zahl, z.B. bis zur Dezimalzahl "4" zählt, wobei des Zählerausgangssignal nur dann "1" ist, wenn der Zählerstand dem Maximalwert "4" entspricht. Bei allen anderen Zählerständen ist das Ausgangssignal des Zählers 31 "0". Der Ausgang des Zählers 31 ist über einen Inverter 33 mit dem zweiten Eingang des UND-Tors 32, sowie über einen Schaltkontakt 34 mit dem einen Eingang eines Summierverstärkers 36 verbunden. Der Ausgang des UND-Tors 32 ist über einen Schaltkontakt 37 mit dem Eingang 35 verbunden. Die Schaltkontakte 34 und 37 werden manuell betätigt und funktionieren komplimentär zueinander, d.h. wenn Kontakt 34 geöffnet, ist Kontakt 37 geschlossen, und umgekehrt.

Die Eingangsleitung 35 des Verstärkers 36 ist außerdem mit dem Steuereingang eines Impulsgenerators 38 verbunden, der für die Dauer des Ansteuersignals eine Dreieckspannung liefert, die über einen Schalter 39 dem zweiten Eingang 40 des Summierverstärkers 36 zugeführt wird. Der Summierverstärker 36 ist ein Leistungsverstärker, dessen Ausgangssignal die Magnetspule 25 steuert.

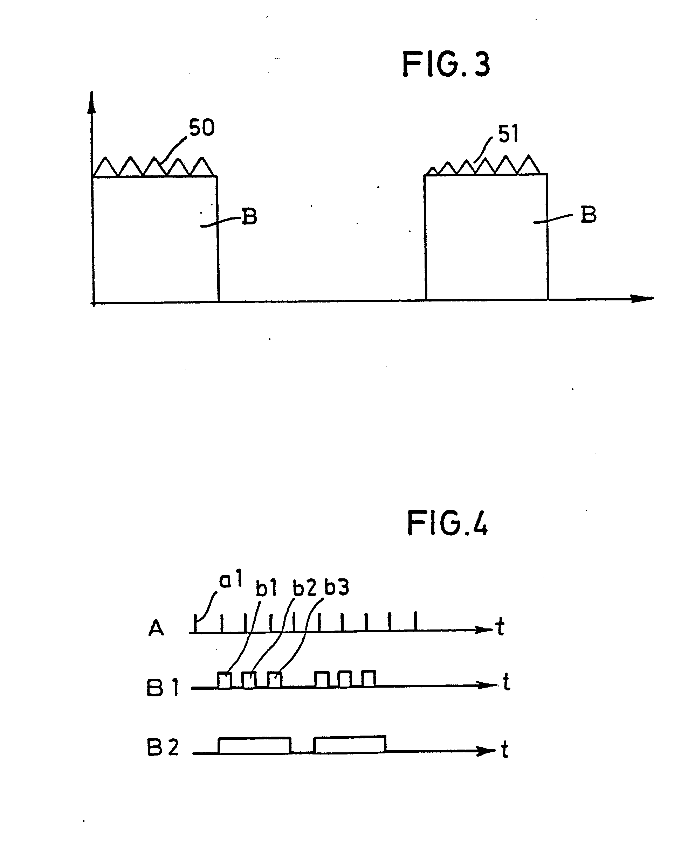

Der in Figur 4 mit A bezeichnete Impulszug kennzeichnet das Ausgangssignal des Schwellwertschalters 28 und gibt die Abstände der Spannungsimpulse 27 des EKG an. Der erste Impuls a1 führt dazu, daß nach der Verzögerungszeit des Verzögerungsgliedes 29 am Ausgang des Impulsformers 30 ein Impuls b1 erzeugt wird. Da der Zählerstand des Zählers 31 zu diesem Zeitpunkt "0" ist, ist über den Inverter 33 das UND-Tor 32 für den Durchgang des Impulses b1 geöffnet, so daß dieser Impuls über den geschlossenen Schalter 37 an den Eingang 35 des Verstärkers 36 gelangt. Die Dauer des Impulses b1 beträgt etwa die Hälfte der Dauer zwischen zwei Impulsen A. Durch geeignete Wahl der Verzögerung kann die Phasenlage des Impulses b1 relativ zu den Impulsen A festgelegt werden. Da der Ausgang des Zählers 31 auch für die nächsten beiden Impulse b2 und b3 "0" ist, werden auch die Impulse b2 und b3 auf den Eingang 35 übertragen. Danach nimmt der Zähler 31 den Wert "4" an, so daß sein Ausgangssignal "1" und das Ausgangssignal des Inverters 33 "0" wird. Dadurch wird das UND-Tor 32 gesperrt, so daß der nächstfolgende Impuls des Impulsformers 30 nicht auf den Ausgang 35 durchgelassen wird. Wie der Impulszug B1 in Figur 4 zeigt, folgt jeweils auf drei Druckimpulse b1, b2 und b3 eine Impulslücke. Die Längen der Druckimpulse, über die die Magnetspule 25 erregt ist, können durch Veränderung der Einstellung des Impulsformers 30 variiert werden.

In Figur 4 zeigt der Impulszug B2 eine andere Betriebsart, bei der der Schalter 34 geschlossen und Schalter 37 geöffnet ist. Bei dieser Betriebsart wird das Ausgangssignal des Inverters 33 direkt dem Eingang 35 zugeführt. Dieses Ausgangssignal B2 besteht aus Impulsen, die über mehrere Takte (z.B. drei Takte) durchgehen und zwischen denen sich eine Impulslücke von einem Takt befindet.

Durch den Impulsgenerator 38 können über den Schalter 19 dem Eingang 40 Wechselsignale 50 zugeführt werden, die beispielsweise sägezahn- oder dreieckförmig sind. Hier sind jedoch auch andere periodische Schwingungsformen möglich. Diese Wechselschwingungen werden im Summierverstärker 36 den Impulsen am Eingang 35 überlagert, so daß sich die in Figur 3 dargestellte Impulsform ergibt, die sich aus dem Grundimpuls B des Eingangs 35 und dem Signal 50 am Eingang 40 zusammensetzt. Die Amplitude des Signals 40 ist wesentlich kleiner als diejenige des Signales B. Jedem Impuls B werden auf diese Weise einige Schwingungen des Signals 50 überlagert. Die gleiche resultierende Impulsform haben dann die Druckimpulse, die in dem Druckbehälter 10 erzeugt und durch den Schlauch 18 an den Dilatationsteil 20 weitergegeben werden, so daß der Dilatationsteil 20 entsprechend den Schwingungen 50 vibriert.

In Figur 3 ist eine weitere Form der Schwingungen 51 dargestellt, wobei diese Schwingungen mit zunehmender Dauer des Impulses B immer größer werden. Auf diese Weise erfolgt eine sich ständig verstärkende Anregung der Gefäßwand durch die Schwingung 51.

**Patentansprüche**

1. Vorrichtung zur Dilatation von Blutgefäßen, mit einem in ein Blutgefäß einsetzbaren, durch Druck aufweitbaren Dilatationskatheter (17) und einem an den Dilatationskatheter (17) anschließbaren, ein Druckfluid enthaltenen Druckbehälter (10), dessen Druck zeitlich gesteuert veränderbar ist und der eine Vorrichtung (23, 24, 25) zur Änderung des das Druckfluid enthaltenen Behältervolumens aufweist, welche zur Erzeugung von Druckimpulsen derart betätigbar ist, daß der Dilatationskatheter (17) abwechselnd das Blutgefäß aufweitet und für den Blutdurchgang freigibt, dadurch gekennzeichnet, daß den Druckimpulsen ein in seiner Amplitude kleinerer, sich periodisch verändernder Wechseldruck (50, 51) überlagert ist.

2. Vorrichtung nach Anspruch 1, dadurch gekennzeichnet, daß die Vorrichtung (23, 24, 25) zur Änderung des Behältervolumens in Abhängigkeit von EKG-Impulsen (27) des Patienten gesteuert ist.

3. Vorrichtung nach Anspruch 2, dadurch gekennzeichnet, daß die Vorrichtung (23, 24, 25) zur Veränderung des Behältervolumens von einem Leistungsverstärker (36) gesteuert ist, der an eine von EKG-Impulsen getriggerte Impulserzeugerschaltung (30, 31) angeschlossen ist.

4. Vorrichtung nach Anspruch 3, dadurch gekennzeichnet, daß den Impulsen der Impulsformerschaltung (30, 31) eine höherfrequente Spannung (50, 51) geringerer Amplitude überlagert ist.

5. Vorrichtung nach Anspruch 4, dadurch gekennzeichnet, daß die Amplitude der höherfrequenten Spannung (51) während eines Impulses (B) der Impulserzeugerschaltung (30, 31) von einem Anfangswert bis zu einem Endwert zu- oder abnimmt.

6. Vorrichtung nach einem der Ansprüche 3 bis 5, dadurch gekennzeichnet, daß die Impulserzeugerschaltung einen Zähler (38) aufweist, der jeden n-ten Impuls unterdrückt, wobei n eine ganze Zahl ist, die größer ist als 1.

7. Vorrichtung nach einem der Ansprüche 3 bis 6, dadurch gekennzeichnet, daß die Impulserzeugerschaltung (30, 31) einen Zähler (31) aufweist, der jeweils über mehrere Ansteuerimpulse hinweg einen Ausgangsimpuls erzeugt, wobei die Ausgangsimpulse durch Impulslücken voneinander getrennt sind.

8. Vorrichtung nach einem der Ansprüche 2 bis 7, dadurch gekennzeichnet, daß die von einem EKG (26) abgeleitete Spannung einer nur auf EKG-Impulse (27) ansprechenden Schwellwertschaltung (28) zugeführt wird und daß der Schwellwertschaltung (28) eine Verzögerungsschaltung (29) mit einstellbarer Verzögerung nachgeschaltet ist.

9. Vorrichtung nach einem der vorhergehenden Ansprüche, dadurch gekennzeichnet, daß der Druckbehälter (10) ein Überdruckventil (15) aufweist, das bei einem höchstzulässigen Druck im Innern des Dilatationskatheters (17) auslöst.

10. Vorrichtung nach Anspruch 3 oder 4, dadurch gekennzeichnet, daß die Impulslänge der Impulse einstellbar ist.

11. Vorrichtung nach einem der vorhergehenden Ansprüche, dadurch gekennzeichnet, daß der Druck in dem Druckbehälter (10) als Eingangsdruck für den Dilatationskatheter (17) mit einem dynamischen Druckaufnehmer simultan registriert wird.

## Revendications

1. Dispositif pour la dilatation de vaisseaux sanguins comprenant un cathéter de dilatation (17) pouvant être dilaté par pression et pouvant être inséré dans un vaisseau sanguin et un réservoir sous pression (10) contenant un fluide sous pression et pouvant être raccordé audit cathéter de dilatation (17), réservoir dont la pression peut être modulée au moyen d'une régulation chrono-dépendante et qui comporte un dispositif (23, 24, 25) destiné à modifier le volume du réservoir contenant le fluide, dispositif manoeuvrable pour la production d'impulsions de pression de telle manière que le cathéter de dilatation (17), alternativement, dilate le vaisseau sanguin et le libère pour permettre le circulation sanguine, caractérisé par le fait qu'aux impulsions de pression est superposée une pression alternée (50, 51) d'amplitude plus faible et variant périodiquement.

2. Dispositif selon la revendication 1, caractérisé par le fait que le dispositif (23, 24, 25) destiné à modifier le volume du réservoir est régulé en fonction des impulsions de l'ECG (27) du patient.

3. Dispositif selon la revendication 2, caractérisé par le fait que le dispositif (23, 24, 25) destiné à modifier le volume du réservoir est régulé par un amplificateur de puissance (36) qui est raccordé à un générateur d'impulsions (30, 31) déclenché par des impulsions de l'ECG.

4. Dispositif selon la revendication 3, caractérisé par le fait qu'aux impulsions du générateur d'impulsions (30, 31) est superposée une tension (50, 51) de fréquence plus élevée et d'amplitude plus faible.

5. Dispositif selon la revendication 4, caractérisé par le fait que l'amplitude de la tension (51) à fréquence plus élevée augmente ou diminue entre une valeur initiale et une valeur finale pendant une impulsion (B) du générateur d'impulsions (30, 31).

6. Dispositif selon l'une des revendications 3 à 5, caractérisé par le fait que le générateur d'impulsions comporte un compteur (38) qui supprime chaque nième impulsion, n étant un nombre entier supérieur à 1.

7. Dispositif selon l'une des revendications 3 à 6, caractérisé par le fait que le générateur d'impulsions (30, 31) comporte un compteur (31) qui produit respectivement sur plusieurs impulsions de commande une impulsion de sortie, lesdites impulsions de sortie étant séparées entre elles par des intervalles sans impulsion.

8. Dispositif selon l'une des revendications 2 à 7, caractérisé par le fait que la tension dérivée d'un ECG (26) est amenée à un circuit à seuil (28) qui ne peut être excité que par les impulsions ECG (27) et par le fait qu'après ce circuit à seuil (28) est monté un circuit de temporisation (29) à délai de temporisation réglable.

9. Dispositif selon l'une quelconque des revendications précédentes, caractérisé par le fait que le réservoir sous pression (10) comporte une soupape de surpression (15) qui se déclenche lorsqu'une pression maximale admissible est atteinte à l'intérieur du cathéter de dilatation (17).

10. Dispositif selon les revendications 3 ou 4, caractérisé par le fait que la longueur des impulsions est réglable.

11. Dispositif selon l'une quelconque des revendications précédentes, caractérisé par le fait que la pression à l'intérieur du réservoir sous pression (10) est enregistrée simultanément par un manomètre enregistreur dynamique afin d'indiquer la pression d'entrée dans le cathéter de dilatation (17).

## Claims

1. Device for the dilatation of blood vessels, comprising a dilatation catheter (17) insertable into a blood vessel and dilatatable by pressure and a pressure container (10) adapted to be connected to the dilatation catheter (17) and containing a pressure fluid, the pressure of the container being changeable by time control and said container including means (23, 24, 25) for changing the container volume containing the pressure fluid which device, for generating pressure pulses, is so operable that the dilatation catheter (17) alternatingly dilates the blood vessel and releases it for the blood passage, characterized in that the pressure pulses are superposed by an alternating pressure (50, 51) having an inferior period and changing periodically.

2. Device according to claim 1, characterized in that means (23, 24, 25) for changing the container volume is controlled responsive to the ECG pulses (27) of the patient.

3. Device according to claim 2, characterized in that the means (23, 24, 25) for changing the container volume is controlled by a power amplifier (36) which is connected to a pulse generating circuit (30, 31) triggered by ECG pulses.

4. Device according to claim 3, characterized in that the pulses of the pulse shaping circuit (30, 31) are superposed by a higher frequency voltage (50, 51) of an inferior amplitude.

5. Device according to claim 4, characterized in that the amplitude of the higher frequency voltage (51) increases or decreases from an initial value to a final value during one pulse (B) of the pulse generating circuit (30, 31).

6. Device according to one of claims 3 to 5, characterized in that the pulse generating circuit contains a counter (38) which suppresses each n-th pulse, n being an integer greater than 1.

7. Device according to one of claims 3 to 6, characterized in that the pulse generating circuit (30, 31) comprises a counter (31) generating along a respective number of triggering pulses one output pulse, the output pulses being mutually separated by pulse gaps.

8. Device according to one of claims 2 to 7, characterized in that the voltage derived from an ECG (26) is supplied to a threshold circuit (28) being only responsive to ECG pulses (27) and that a delay circuit (29) having an adjustable time large is connected behind the threshold circuit (28).

9. Device according to one of the preceding claims, characterized in that the pressure container (10) includes a pressure control value (15) which is actuated in case of a maximum permissible pressure inside the dilatation catheter (17).

10. Device according to claim 3 or 4, characterized in that the pulse length of the pulses is adjustable.

11. Device according to one of the preceding claims, characterized in that the pressure in the pressure container (10) is registered simultaneously as input pressure for the dilatation catheter (17) by means of a dynamic pressure sensor.

FIG.1

FIG.2

# FIG. 3

50

51

B

B

# FIG. 4

a1  b1  b2 b3

A ———————————————→ t

B 1 ———————————————→ t

B 2 ———————————————→ t